**Europäisches Patentamt**

**European Patent Office**

**Office européen des brevets**

(19)

(11) Veröffentlichungsnummer : **0 416 583 B1**

## (12) EUROPÄISCHE PATENTSCHRIFT

(45) Veröffentlichungstag der Patentschrift :
23.12.92 Patentblatt 92/52

(51) Int. Cl.⁵ : **C07C 229/30**, C07C 227/16

(21) Anmeldenummer : **90117091.0**

(22) Anmeldetag : **05.09.90**

(54) **Verfahren zur Herstellung von Crotonobetainhydrochlorid.**

(30) Priorität : **07.09.89 CH 3253/89**

(43) Veröffentlichungstag der Anmeldung :
**13.03.91 Patentblatt 91/11**

(45) Bekanntmachung des Hinweises auf die
Patenterteilung :
**23.12.92 Patentblatt 92/52**

(84) Benannte Vertragsstaaten :
**AT BE CH DE DK ES FR GB IT LI NL SE**

(56) Entgegenhaltungen :
**EP-A- 0 244 370**

(56) Entgegenhaltungen :
**CHEMICAL ABSTRACTS, Band 59, 1963, Spalte 6248, Zusammenfassung H, Columbus, Ohio, US; F. BINON et al.: "Derivatives ofcarnitine and crotonebetaine", & BULL. SOC. CHIM. BELGES 72, 166-77 (1963)**
**CHEMICAL ABSTRACTS, Band 45, 1951, Spalte 1013, Zusammenfassung e, Columbus, Ohio, US; F. BERGEL et al.: "Syntheses ofneurotropic and musculotropic stimulators and inhibitors. III. Derivatives of unsaturated omega-betaines", & J. CHEM. SOC.1950, 1439-43**

(73) Patentinhaber : **LONZA AG**
**Gampel/Wallis (CH)**

(72) Erfinder : **Worsch, Detlev, Dr.**
**Faxugrundstrasse 30**
**Brigerbad (Kanton Wallis) (CH)**

(74) Vertreter : **Weinhold, Peter, Dr. et al**
**Patentanwälte Dipl.-Ing. G. Dannenberg Dr. P. Weinhold Dr. D. Gudel Dipl.-Ing. S. Schubert Dr. P. Barz Siegfriedstrasse 8**
**W-8000 München 40 (DE)**

**Beschreibung**

Die Erfindung betrifft ein neues Verfahren zur Herstellung von Crotonobetainhydrochlorid aus Carnitinhydrochlorid, insbesondere aus racemischem Carnitinhydrochlorid oder aus D-Carnitinhydrochlorid.

Crotonobetainhydrochlorid wird u.a. als Ausgangsprodukt in der mikrobiologischen Synthese von L-Carnitin eingesetzt, vgl. z.B. EP-A 0 158 194 oder EP-A 0 122 794.

Es ist gemäss Chemical Abstracts Vol.59, 6248 (Binon et al) bekannt, Crotonobetainhydrochlorid in 78%iger Ausbeute aus D,L-Carnitinchlorid durch Umsetzung mit konz. Schwefelsäure bei 130°C, anschliessender Ausfällung mit Aceton und Behandlung mit Bariumchlorid zu gewinnen.
Ein grosser Nachteil dieses Verfahrens ist neben der Handhabung von konz. Schwefelsäure das anfallende und nicht rezirkulierbare Bariumsulfat.

Im weiteren stellt sich bei der klassischen chemischen Herstellung von L-Carnitin durch Racemattrennung, z.B. nach DD 23.217, zwangsläufig das Problem, dass D-Carnitin anfällt, das bisher keiner weiteren Verwendung zuführbar war.

Es bestand daher die Aufgabe, ein Verfahren zu finden, das die genannten Nachteile ausschliesst.

Die Aufgabe konnte gelöst werden mit einem Verfahren nach Patentanspruch 1.

Darin wird wahlweise racemisches Carnitinhydrochlorid, D-Carnitinhydrochlorid oder auch L-Carnitinhydrochlorid mit 1,5 bis 15 mol Acetanhydrid in Gegenwart eines sauren Katalysators bei einer Temperatur von 90 bis 130°C umgesetzt. Vorzugsweise wird selbstverständlich das in der klassischen L-Carnitinsynthese anfallende D-Carnitin oder das kostengünstige D,L-Carnitin eingesetzt.

Vorzugsweise wählt man die Acetanhydridmengen im Bereich von 1,8 bis 2 mol.

Als saurer Katalysator wird vorzugsweise p-Toluolsulfonsäure in einer Menge von 0,5 bis 2 Gew.%, bezogen auf eingesetztes Carnitinhydrochlorid, eingesetzt.

Die Reaktionstemperatur liegt vorzugsweise zwischen 110 und 125°C, besonders bevorzugt zwischen 115 und 120°C. Bei dieser Temperatur ist die Reaktion in der Regel nach ca. 2 Stunden beendet.

Durch Zugabe eines niederen aliphatischen Alkohols, vorzugsweise Ethanol, zum warmen, vorzugsweise 70 bis 80°C warmen Reaktionsgemisch und durch anschliessendes Abkühlen kann auf vorteilhafte Weise das Crotonobetainhydrochlorid ausgefällt und mit einer bereits hohen Reinheit isoliert werden.

Nach dem erfindungsgemässen Verfahren können Ausbeuten von über 70% erreicht werden.

Das Crotonobetainhydrochlorid kann auf übliche Weise, z.B. mittels Elektrodialyse, entsalzt und in das Crotonobetain überführt werden.

Das nach dem erfindungsgemässen Verfahren hergestellte Produkt eignet sich in besonderer Weise zum Einsatz in der mikrobiologischen Herstellung von L-Carnitin.

Beispiel

25,0 g (0,125 mol) D,L-Carnitinhydrochlorid, 0,25 g (1,5 mmol) p-Toluolsulfonsäure und 25,0 g (0,245 mol) Acetanhydrid wurden 2 h auf 120°C erhitzt. Man liess die dunkel gefärbte Lösung auf ca. 80°C abkühlen und gab 20 ml Ethanol hinzu. Danach liess man langsam weiter abkühlen, wobei das Produkt auszufallen begann. Mit einem Eiswasserbad wurde die Suspension auf ca. 6°C abgekühlt, abgesaugt und mit wenig kaltem Ethanol nachgewaschen. Das Produkt wurde unter Vakuum getrocknet.
Man erhielt 17,4 g beigefarbenes Pulver mit einem Gehalt von 96% (HPLC). Dies entsprach eines Ausbeute von 73,5%.
$^1$H-NMR, dmso d$_6$:
  3,19 (s,-CH$_3$, 9H)
  4,38 (d 8Hz, -CH$_2$, 2H)
  6,33 (d 15Hz, CH=, 1H)
  6,90 (dt 15Hz, CH=, 1H)
  13,0 (s breit, -COOH, 1H)

**Patentansprüche**

1.  Verfahren zur Herstellung von Crotonobetainhydrochlorid, dadurch gekennzeichnet, daß Carnitinhydrochlorid als Racemat oder in Form seiner Enantiomere mit 1,5 bis 15 mol Acetanhydrid in Gegenwart eines sauren Katalysators bei einer Temperatur zwischen 90 und 130°C umgesetzt wird.

2.  Verfahren nach Patentanspruch 1, dadurch gekennzeichnet, dass 1,8 bis 2 mol Acetanhydrid eingesetzt werden.

3.  Verfahren nach mindestens einem der Patentansprüche 1 bis 2, dadurch gekennzeichnet, dass als saurer Katalysator p-Toluolsulfonsäure in einer Menge von 0,5 bis 2 Gew.%, bezogen auf eingesetztes Carnitinhydrochlorid, verwendet wird.

4.  Verfahren nach mindestens einem der Patentansprüche 1 bis 3, dadurch gekennzeichnet, dass in einem Temperaturbereich von 110 bis 125°C gearbeitet wird.

5.  Verfahren nach mindestens einem der Patentansprüche 1 bis 4, dadurch gekennzeichnet, dass nach beendeter Reaktion durch Zugabe eines niederen aliphatischen Alkohols zum Reaktionsgemisch und durch darauf folgendes Abkühlen das Crotonobetainhydrochlorid ausgefällt und isoliert wird.

## Claims

1. Process for the preparation of crotonobetaine hydrochloride, characterized in that carnitine hydrochloride as a racemate or in the form of its enantiomers is reacted with 1.5 to 15 moles of acetoanhydride in the presence of an acidic catalyst at a temperature between 90 and 130°C.

2. Process according to patent claim 1, characterized in that 1.8 to 2 moles of acetoanhydride are used.

3. Process according to at least one of patent claims 1 to 2, characterized in that as acidic catalyst p-toluene sulfonic acid is used in an amount of from 0.5 to 2 weight%, based on the carnitine hydrochloride employed.

4. Process according to at least one of patent claims 1 to 3, characterized in that one operates in a temperature range of from 110 to 125°C.

5. Process according to at least one of patent claims 1 to 4, characterized in that, after the reaction has been completed the crotonobetaine hydrochloride is precipitated by the addition of a lower aliphatic alcohol to the reaction mixture and subsequent cooling and then isolated.

## Revendications

1. Procédé pour la préparation de chlorhydrate de crotonobétaïne, caractérisé en ce que le chlorhydrate de carnitine est mis en réaction sous forme de racémate ou sous forme de son énantiomère avec 1,5 jusqu'à 15 moles d'anhydride acétique en présence d'un catalyseur acide à une température entre 90 et 130°C.

2. Procédé selon la revendication 1, caractérisé en ce que l'on met en oeuvre 1,8 jusqu'à 2 moles d'anhydride acétique.

3. Procédé selon au moins l'une des revendications 1 à 2, caractérisé en ce qu'en tant que catalyseur acide on utilise l'acide de p-toluolsulfonique dans une quantité de 0,5 à 2 % en poids rapportés au chlorhydrate de carnitine mis en oeuvre.

4. Procédé selon au moins l'une des revendications 1 à 3, caractérisé en ce que l'on travaille dans une plage de températures de 110 à 125°C.

5. Procédé selon au moins l'une des revendications 1 à 4, caractérisé en ce qu'après achèvement de la réaction, par addition d'un alcool aliphatique inférieur au mélange réactionnel et par refroidissement successif, on fait précipiter et on isole le chlorhydrate de crotonobétaïne.